# EUROPEAN PATENT SPECIFICATION

(11) **EP 2 363 478 B1**
(45) Date of publication and mention of the grant of the patent: **24.07.2019**
(21) Application number: 10010661.6
(22) Date of filing: 19.04.2000
(51) Int. Cl.: C12N 15/11, C12N 15/63, C12N 15/70, C12N 15/86, C07K 14/01, C07K 14/08, A61K 48/00, A61K 38/16, A61P 35/00

(54) **Methods and compositions for inhibiting the function of polynucleotide sequences**
Verfahren und Zusammensetzungen zur Hemmung der Funktion der Polynukleotide Sequenzen
Procédés et compositions pour inhiber la fonction de séquences de polynucléotides

(30) Priority: 21.04.1999 US 130377 P
(43) Date of publication of application: 07.09.2011
(62) Divisional of application: 00926145.4
(73) Proprietor: Alnylam Pharmaceuticals, Inc., Cambridge, MA 02142 (US)
(72) Inventor: Pachuk, Catherine, Cambridge, MA 92142 (US); Satishchandran, Chandrasekhar, Landsdale, PA 19446 (US)
(74) Representative: Mewburn Ellis LLP

(56) References cited:
- WO-A-00/44914
- WO-A-95/27783
- WO-A-97/07825
- WO-A-97/34638
- WO-A-98/05770
- WO-A-99/53050
- US-A- 4 891 315
- MONTGOMERY M K ET AL: "Double-stranded RNA as a mediator in sequence-specific genetic silencing and co-suppression", TRENDS IN GENETICS,NL,ELSEVIER SCIENCE PUBLISHERS B.V. AMSTERDAM, vol. 14, no. 7, 1 July 1998 (1998-07-01), pages 255-258, XP004124680, ISSN: 0168-9525
- PLAYER MARK R ET AL: "Targeting HIV mRNA for degradation: 2,5-A antisense chimeras as potential chemotherapeutic agents for AIDS.", NUCLEOSIDES & NUCLEOTIDES, vol. 16, no. 7-9, July 1997 (1997-07), pages 1221-1222, XP000952972, ISSN: 0732-8311
- MIKITA T ET AL: "EFFECTS OF ARABINOSYLCYTOSINE-SUBSTITUTED DNA ON DNA/RNA HYBRID STABILITY AND TRANSCRIPTION BY T7 RNA POLYMERASE", BIOCHEMISTRY,US,AMERICAN CHEMICAL SOCIETY. EASTON, PA, vol. 33, 1994, pages 9195-9208, XP000882431, ISSN: 0006-2960
- YUKIKO TONE ET AL: "STRUCTURE AND CHROMOSOMAL LOCATION OF THE MOUSE INTERLEUKIN-12 P35 AND P40 SUBUNIT GENES", EUROPEAN JOURNAL OF IMMUNOLOGY,DE,WEINHEIM, vol. 26, no. 6, 1 June 1996 (1996-06-01), pages 1222-1227, XP000612644, ISSN: 0014-2980
- JAEGER J A ET AL: "IMPROVED PREDICTIONS OF SECONDARY STRUCTURES FOR RNA", PROCEEDINGS OF THE NATIONAL ACADEMY OF SCIENCES OF THE UNITED STATES, vol. 86, no. 20, 1989, pages 7706-7710, XP000953154, 1989 ISSN: 0027-8424
- WATERHOUSE ET AL: "Virus resistance and gene silencing in plants can be induced by simultaneous expression of sense and antisense RNA", PROCEEDINGS OF THE NATIONAL ACADEMY OF SCIENCES OF USA,NATIONAL ACADEMY OF SCIENCE. WASHINGTON,US, vol. 95, November 1998 (1998-11), pages 13959-13964, XP002114472, ISSN: 0027-8424
- NELLEN W ET AL: "WHAT MAKES AN MRNA ANTI-SENSE-ITIVE?", TIBS TRENDS IN BIOCHEMICAL SCIENCES,EN,ELSEVIER PUBLICATION, CAMBRIDGE, vol. 18, no. 11, 1 November 1993 (1993-11-01), pages 419-423, XP002049385, ISSN: 0968-0004
- FIRE A ET AL: "Potent and specific genetic interference by double-stranded RNA in Caenorhabditis elegans", NATURE,GB,MACMILLAN JOURNALS LTD. LONDON, vol. 391, 19 February 1998 (1998-02-19), pages 806-811, XP002095876, ISSN: 0028-0836
- WIANNY F ET AL: "Specific interference with gene function by double-stranded RNA in early mouse development", NATURE CELL BIOLOGY,GB,MACMILLAN PUBLISHERS, vol. 2, no. 2, February 2000 (2000-02), pages 70-75, XP002138445, ISSN: 1465-7392
- FIRE A ET AL: "Potent and specific genetic interference by double-stranded RNA in Caenorhabditis elegans", NATURE, NATURE PUBLISHING GROUP, UNITED KINGDOM, vol. 391, 19 February 1998 (1998-02-19), pages 806-811, XP002095876, ISSN: 0028-0836, DOI: 10.1038/35888

## Description

### Field of the Invention

The present invention relates to polynucleotide compositions which have an inhibitory or other regulatory effect upon the function of certain target polynucleotide sequences present in a mammalian cell, for use in a method of treatment.

### Background of the Invention

Polynucleotide compositions have been described for pharmaceutical uses, primarily for treatment or prophylaxis of disease in mammals, as well as in research in such fields. Specifically a great deal of activity presently surrounds the use of polynucleotide compositions in the treatment of pathogenic extracellular and intracellular infections, such as viral, bacterial, fungal infections, and the like. As one example, DNA vaccines are described to deliver to a mammalian cell *in vivo* an agent which combats a pathogen by harnessing the mammalian immune system. Thus, such vaccines are designed to express, for example, a viral protein or polypeptide, and elicit a humoral or cellular immune response upon challenge by the infective agent. Gene therapy vectors, on the other hand, are polynucleotide compositions generally designed to deliver to a mammalian cell a protein which is either not expressed, expressed improperly or underexpressed in a mammal. Such vectors frequently must address species specific immune responses to the those polynucleotide sequences that are recognized as antigenic or which evoke an unwanted cellular immune response.

Still other therapeutic uses of polynucleotide compositions are for the delivery of missing or underexpressed proteins to a diseased mammalian patient. Furthermore, polynucleotides are useful themselves as *in vivo* reagents, in diagnostic/imaging protocols, as reagents in gene therapy, in antisense protocols and in vaccine applications or otherwise as pharmaceuticals used to treat or prevent a variety of ailments such as genetic defects, infectious diseases, cancer, and autoimmune diseases. Polynucleotides are also useful as *in vitro* reagents in assays such as biological research assays, medical, diagnostic and screening assays and contamination detection assays.

A host of problems well-known to the art has prevented the numerous polynucleotide compositions from becoming widely accepted as useful pharmaceutics. Thus, there are few such DNA vaccines or therapeutics which have yet been accepted by the medical community for the treatment of disease in mammals.

Phenomena have been observed in plants and nematodes that are mediated by polynucleotide compositions, and are referred to as post-transcriptional gene silencing and transcriptional silencing. This phenomenon demonstrates that transfection or infection of a plant, nematode or Drosophila with a virus, viroid, plasmid or RNA expressing a polynucleotide sequence having some homology to a regulatory element, such as a promoter or a native gene or a portion thereof already expressed in that cell, can result in the permanent inhibition of expression of both the endogenous regulatory element or gene and the exogenous sequence. This silencing effect was shown to be gene specific. See, for example, L. Timmons and A. Fire, Nature, 395:354 (Oct. 29, 1998); A. Fire et al, Nature, 391:806-810 (Feb. 19, 1998); R. Jorgensen et al, Science, 279:1486-1487 (March 6, 1998)], and Montgomery et al, Trends in Genetics vol. 14(7) 1998. A DNA plasmid encoding a full-length pro-alpha 1 collagen gene was transiently transfected into a rodent fibroblast tissue cell line and a "silencing" effect on the native collagen gene and the transiently expressed gene observed [Bahramian and Zarbl, Mol. Cell. Biol., 19(1):274-283 (Jan. 1999)].

See, also, International Patent Application No. WO98/05770, published February 12, 1998, which relates to gene inhibition by use of an antisense RNA with secondary structures, and/or in combination with double stranded RNAse. International Patent Application No. WO99/53050, published October 21, 1999, also relates to reducing phenotypic expression of a nucleic acid, particularly in plant cells, by introducing chimeric genes encoding sense and anti-sense RNA molecules.
WO 95/27783 describes a method of inhibiting human immunodeficiency virus type 1 (HIV-1) in a mammal using mammalian cells, particularly, human CD4 containing lymphocytes, which express chimeric RNA molecules containing HIV-1 Ψ signal and/or Gag coding sequences in an antisense orientation.

There exists a need in the art for polynucleotide compositions and methods of using same to inhibit the function of polynucleotide sequences which are disease-causing in mammals, such as polynucleotide sequences essential for the replication of viruses and other intracellular pathogens in mammalian cells, or sequences of extracellular mammalian pathogens, or sequences of tumor antigens which mediate the spread of cancer in a mammal, and the like, without adversely affecting essential gene sequences in the mammal.

### Summary of the Invention

In one aspect, the invention provides a composition for use in a method of treatment comprising inhibiting the function of a target polynucleotide sequence in a mammalian cell, wherein the target polynucleotide sequence is a non-coding sequence of a target gene. The composition comprises an agent that provides to a mammalian cell an at least partially double-stranded RNA molecule wherein said at least partially double-stranded RNA molecule is less than about 1,000 nucleotides in length and contains a double-stranded sequence of at least 11 to 30 nucleotides, wherein said at least partially double stranded RNA comprises a sense sequence of a target polynucleotide and an antisense sequence thereto, and wherein said target gene is a naturally occuring mammalian sequence or a heterologous sequence present due to mammalian intracellular pathogenic infection. For instance, the target polynucleotide sequence can be a polynucleotide sequence, e.g., of a virus or other intracellular pathogen or a polynucleotide sequence of a cancer antigen or of an essential tumorigenic regulatory sequence.

This RNA molecule preferably does not produce a functional protein. This RNA molecule is preferably substantially non-homologous to naturally-occurring, essential mammalian polynucleotide sequences. In one embodiment, the agent of this composition is an RNA molecule made by enzymatic synthetic methods or chemical synthetic methods *in vitro.* In another embodiment, the RNA molecule may be generated in a recombinant culture, e.g., bacterial cells, isolated therefrom, and used in the methods discussed below. In another embodiment the agent of this composition generates the RNA molecule *in vivo* after delivery to the mammalian cell.

Also described herein is a pharmaceutical composition comprising one or more of the compositions described immediately above and specifically hereinbelow, and an optional second agent that facilitates polynucleotide uptake in a cell, in a pharmaceutically acceptable carrier. Such compositions are useful for treating intracellular pathogenic infections, such as viruses. Other such compositions are useful for treating certain cancers. Other such compositions are useful for treating certain extracellular pathogens. Still other such compositions are useful for treating any disease or disorder wherein inhibiting the function of a polynucleotide sequence in a mammal is desirable for therapy or vaccine use.

The method of treatment may be a method for treating a viral infection in a mammal by administering to the mammal one or more of the above-described compositions wherein the target polynucleotide is a virus polynucleotide sequence necessary for replication and/or pathogenesis of the virus in an infected mammalian cell, along with an optional second agent that facilitates polynucleotide uptake in a cell, in a pharmaceutically acceptable carrier. This composition is administered in an amount effective to reduce or inhibit the function of the viral sequence in the cells of the mammal.

The method of treatment may be a method for preventing a viral infection in a mammal by administering to the mammal one or more of the above-described compositions wherein the target polynucleotide is a virus polynucleotide sequence necessary for replication and/or pathogenesis of the virus in an infected mammalian cell, with an optional second agent that facilitates polynucleotide uptake in a cell, in a pharmaceutically acceptable carrier. This composition is administered in an amount effective to reduce or inhibit the function of the viral sequence upon subsequent introduction of the virus into the mammalian cells.

The method of treatment may be a method for treatment or prophylaxis of a virally induced cancer in a mammal by administering to the mammal one or more of the above described compositions in which the target polynucleotide is a regulatory sequence, which sequence function is required for the maintenance of the tumor in the mammal. The compositions can contain an optional second agent that facilitates polynucleotide uptake in a cell, and a pharmaceutically acceptable carrier. The composition is administered in an amount effective to reduce or inhibit the function of the tumor-maintaining sequence in the mammal.

The method of treatment may be a method for the treatment or prophylaxis of infection of a mammal by an intracellular pathogen. The mammal is administered one or more of the compositions herein described wherein the target polynucleotide is a polynucleotide sequence of the intracellular pathogen necessary for replication and/or pathogenesis of the pathogen in an infected mammalian cell. The composition is administered with an optional second agent that facilitates polynucleotide uptake in a cell, in a pharmaceutically acceptable carrier, in an amount effective to reduce or inhibit the function of the sequence in the mammal.

The method of treatment may be a method of treatment or prophylaxis of cancer in a mammal. The mammal is administered one or more of the above-described compositions, wherein the target polynucleotide is a polynucleotide sequence of an abnormal cancer-causing gene or non-expressed regulatory sequence in a mammal, which also possesses a normal copy of the gene or regulatory sequence. According to this aspect, the differences between the abnormal sequence and the normal sequence are differences in polynucleotides. The composition is administered with an optional second agent that facilitates polynucleotide uptake in a cell, in a pharmaceutically acceptable carrier, and in an amount effective to reduce or inhibit the function of the abnormal sequence in the mammal.

The method of treatment may be a method for treating a disease or disorder in a mammal comprising administering to the mammal having a disease or disorder characterized by expression of polynucleotide product not found in a healthy mammal, one or more of the compositions as above described, in which the target polynucleotide sequence is the polynucleotide sequence which expresses that polynucleotide product or a non-expressed regulatory sequence essential to the expression of that product. The composition is administered with or without a second agent that facilitates polynucleotide uptake in a cell, and in a pharmaceutically acceptable carrier, in an amount effective to reduce or inhibit the function of the target polynucleotide product or regulatory sequence in the cells of the mammal.

Other aspects of the invention are described further in the following detailed description of the preferred embodiments thereof.

### Brief Description of the Figures

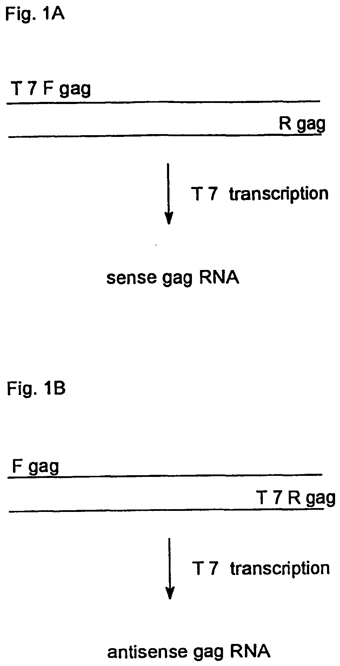

Fig. 1A is an illustration of a PCR product generated using the bacteriophage T7 RNA polymerase promoter- forward gag primer (T7F) and reverse gag (R) primer. Transcription from this PCR template, using T7 RNA polymerase generates an RNA sequence gag sense strand.
Fig. 1B is an illustration of a PCR product generated using a forward gag primer (F) and T7 promoter reverse gag (T7R) primer. Transcription of this template using a T7 RNA polymerase generates an RNA sequence gag antisense strand. Use of both the template of Fig. 1A and the template of Fig. 1B yields double stranded gag RNA sequence.

### Detailed Description of the Invention

The present invention provides novel polynucleotide compositions for use in methods for therapy and prophylaxis, in diseases and disorders which afflict mammalian species, in which the goal is to reduce or inhibit the function of a selected target polynucleotide sequence. These compositions and methods further enable the harnessing of the molecular mechanisms of the cell to accomplish therapeutic goals without requiring any stimulation of the immune system of the mammal involved.

As used herein, the phrases "target" or "target polynucleotide sequence" refer to any sequence present in a mammalian cell or in a mammalian organism, whether a naturally occurring, and possibly defective, mammalian polynucleotide sequence or a heterologous sequence present due to an intracellular pathogenic infection or a disease, which polynucleotide sequence has a function that is desired to be reduced or inhibited. The target sequence of the present invention is non-coding, but may have a regulatory function. One target polynucleotide sequence is a virus polynucleotide sequence necessary for replication and/or pathogenesis of the virus in an infected mammalian cell. Another embodiment of a target polynucleotide sequence is a tumor antigen or functional fragment thereof, or a non-expressed regulatory sequence of a virus-induced cancer, which sequence is required for the maintenance of the tumor in the mammal. Still another embodiment of a target polynucleotide sequence is a polynucleotide sequence of an intracellular pathogen necessary for replication and/or pathogenesis of that pathogen in an infected mammal. Yet another embodiment of a target polynucleotide sequence is a polynucleotide sequence of an abnormal cancer-causing gene (or a non-expressed regulatory sequence) in a mammal which also possesses a normal copy of the gene or sequence. The differences between the abnormal sequence and the normal sequence are differences at the polynucleotide sequence level. Such an abnormal sequence can be, for example, a fusion of two normal genes, and the target sequence can be the sequence which spans that fusion. An example of a fusion is the BCR-abl gene sequence characteristic of certain leukemias. The term "gene" is intended to include any target sequence intended to be "silenced", whether or not transcribed and/or translated, including regulatory sequences, such as promoters.

The terms "mammal" or "mammalian" are intended to encompass their normal meaning. While the invention is most desirably intended for efficacy in humans, it may also be employed in domestic mammals such as canines, felines, and equines, as well as in mammals of particular interest, e.g., zoo animals, farmstock and the like.

### A. The Compositions of the Invention

A composition for inhibiting the function of a target polynucleotide sequence in a mammalian cell, described herein comprises an agent that provides to a mammalian cell an at least partially double-stranded RNA molecule. In general, the term "RNA" may also include RNA-DNA hybrids, except where specified otherwise, e.g., where a 2' -OH group of ribose is required for a particular linkage. The RNA molecule comprises a polynucleotide sequence of at least about 200 nucleotides in length. Importantly, this polynucleotide sequence of the RNA molecule is substantially homologous to the target polynucleotide sequence. This polynucleotide sequence also preferably contains exon sequences or portions thereof Desirably, the polynucleotide sequence does not contain intron sequences. Preferably, the RNA molecule does not produce a functional protein, and more preferably, it is not translated. The polynucleotide sequence of the RNA molecule is preferably substantially non-homologous to any naturally occurring, normally functioning, essential mammalian polynucleotide sequence. The polynucleotide sequences described herein may employ a multitarget or polyepitope approach, e.g., encoding sequences of more than one gene of a single target pathogen or against more than one target pathogen, or other category of target desired to be silenced.

Described herein is an "at least partially double stranded RNA molecule" which includes an RNA polynucleotide sequence of between about 100 to 10,000 polynucleotides in length. At present the sequence is most desirably at least 200 polynucleotides in length, but it can range in one example from 200 to 8000 polynucleotides in length. In another example the RNA molecule can be less than 7500 polynucleotides in length. In still another example the RNA molecule can have a sequence length less than about 5000 polynucleotides. In yet another example the RNA molecule can have a sequence length less than about 2000 polynucleotides. The RNA molecule for use in the present invention has a sequence length less than about 1000 polynucleotides. In yet another embodiment the RNA molecule can have a sequence length less than about 750 polynucleotides.

Minimally, to keep the RNA molecule stable, it has a minimum of 11 to 30 nucleotides involved in a double-stranded sequence, depending upon the composition of the polynucleotide sequence and a ΔG of about -9.2 kcal/mol. As known in the art, ΔG defines the state of minimal external energy required to keep a molecular configuration stable [see, e.g., Jaeger et al, Proc. Natl. Acad. Sci., USA, 20:7706-7710 (1989); and Soler and Jankowski, Math. Biosci., 2:167-190 (1991)]. Based on this minimum, preferably at least 10% of this partially double-stranded RNA molecule sequence is double-stranded. Alternatively, the double stranded portion of these RNA molecules can be at least 30% of the sequence. In another embodiment, the double stranded portion of these molecules can be at least 50% of the sequence. In still another embodiment, the double stranded portion of these molecules can be at least 70% of the sequence. In another embodiment, the double stranded portion of these molecules can be at least 90% of the sequence. In another embodiment, the entire sequence can be double stranded. Alternatively, the double-stranded portion of these molecules can occur at either or both termini, or in some middle portion of the sequence, if the molecule is linear. Similarly, the double-stranded portion can be in any location if the molecule is circular. In certain embodiments of the present invention, the double-stranded portion of the RNA molecule becomes double-stranded only when the molecule is in the mammalian cell. In still other embodiment of this invention, the partially double-stranded molecule is an RNA/DNA hybrid, for example, a single chain containing RNA and DNA, prepared *in vitro*; or a duplex of two such single chains or portions thereof. In yet another embodiment, the RNA molecule, made *in vivo* or *in vitro,* is a duplex comprised of an RNA single strand and a DNA single strand.

The partially double-stranded RNA molecule polynucleotide sequence must be substantially homologous to the target polynucleotide sequence in order to effectively reduce or inhibit the function thereof. The necessary homology may be suitably defined by use of a computer algorithm. As known in the art, "homology" or "identity" means the degree of sequence relatedness between two polypeptide or two polynucleotide sequences as determined by the identity of the match between two lengths of such sequences. Both identity and homology can be readily calculated by methods extant in the prior art [See, e.g., COMPUTATIONAL MOLECULAR BIOLOGY, Lesk, A.M., ed., Oxford University Press, New York, (1988); BIOCOMPUTING: INFORMATICS AND GENOME PROJECTS, Smith, D.W., ed., Academic Press, New York, (1993); COMPUTER ANALYSIS OF SEQUENCE DATA, PART I, Griffin, A.M., and Griffin, H.G., eds., Humana Press, New Jersey, (1994); SEQUENCE ANALYSIS IN MOLECULAR BIOLOGY, von Heinje, G., Academic Press, (1987); and SEQUENCE ANALYSIS PRIMER, Gribskov, M. and Devereux, J., eds., M Stockton Press, New York, (1991)]. While there exist a number of methods to measure identity and homology between two polynucleotide sequences, the terms "identity", "similarity" and homology are well known to skilled artisans [H. Carillo and D. Lipton, SIAM J. Applied Math., 48:1073 (1988)]. Methods commonly employed to determine identity or homology between two sequences include, but are not limited to, those disclosed in Guide to Huge Computers, Martin J. Bishop, ed., Academic Press, San Diego, 1994, and H. Carillo and D. Lipton, SIAM J. Applied Math., 48:1073 (1988). Preferred methods to determine identity or homology are designed to give the largest match between the two sequences tested. Methods to determine identity and similarity are codified in computer programs. Preferred computer program to determine identity and homology between two sequences include, but are not limited to, the algorithm BESTFIT from the GCG program package [J. Devereux et al., Nucl. Acids Res., 12(1):387 (1984)], the related MACVECTOR program (Oxford), and the FASTA (Pearson) programs. For instance, searches for sequence similarities in databases between significant naturally occurring mammalian polynucleotide sequences and target polynucleotide sequences enable the design of suitable RNA molecules desired for use in the invention. The algorithm and/or the degree of homology necessary for any particular RNA molecule may be selected by one of skill in the art, depending on the identity of the target, and/or the closeness of homology of the target sequence to any naturally occurring mammalian sequence, which is desired to be left functioning normally after use of the methods of this invention.

In one preferred embodiment, the RNA polynucleotide sequence desirably has an overall homology of at least 10% to the target sequence and contains at least one segment (window) of 30 contiguous nucleotides with a homology in that window of at least 50% to a similar 30 nts region of the target sequence, using the MACVECTOR program with a default annealing temperature of 37°C. In another embodiment, the RNA polynucleotide sequence desirably has an overall homology of at least 30% to the target sequence and contains at least one window of 30 contiguous nucleotides with a homology in that window of at least 50% to a similar 30 nts region of the target sequence. In another embodiment, the RNA polynucleotide sequence desirably has an overall homology of at least 50% to the target sequence and contains at least one window of 30 contiguous nucleotides with a homology in that window of at least 50% to a similar 30 nts region of the target sequence. In another embodiment, the RNA polynucleotide sequence desirably has an overall homology of at least 70% to the target sequence and contains at least one window of 30 contiguous nucleotides with a homology in that window of at least 50% to a similar 30 nts region of the target sequence. In another embodiment, the RNA polynucleotide sequence desirably has an overall homology of at least 90% to the target sequence and contains at least one window of 30 contiguous nucleotides with a homology in that window of at least 50% to a similar 30 nts region of the target sequence.

In still another embodiment, the RNA polynucleotide sequence desirably has an overall homology of at least 1 0% to the target sequence and contains at least one windows of 30 contiguous nucleotides with a homology in that window of at least 70% to a similar 30 nts region of the target sequence. In another embodiment, the RNA polynucleotide sequence desirably has an overall homology of at least 10% to the target sequence and contains at least one segment (window) of 30 contiguous nucleotides with a homology in that window of at least 90% to a similar 30 nts region of the target sequence.

In yet another embodiment, the RNA polynucleotide sequence desirably has an overall homology of at least 10% to the target sequence and contains at least two windows of 30 contiguous nucleotides with a homology in the windows of at least 50% to similar 30 nts regions of the target sequence. Other embodiments of this formula can be developed by one of skill in the art.

In a second preferred embodiment, the RNA polynucleotide sequence desirably has an overall homology of at least 10% to the target sequence and contains at least one segment (window) of 5 contiguous nucleotides with absolute homology in that window to a 5 nts region of the target sequence, using the MACVECTOR program with a default annealing temperature of 37°C. In another variant of this embodiment, the RNA polynucleotide sequence desirably has an overall homology of at least 30% to the target sequence and contains at least one window of 5 contiguous nucleotides with absolute homology to a 5 nts region of the target sequence. In another embodiment, the RNA polynucleotide sequence desirably has an overall homology of at least 50% to the target sequence and contains the above described 5 nt absolutely homologous window. Other variants of this embodiment can be developed by one of skill in the art.

The presence of the windows referred to in the formulae above permits the overall homology of the remainder of the sequence to be low; however it is anticipated that a low overall homology is likely to affect the dosage of the therapeutic compositions described below adversely. An increase in the number of such windows in the RNA polynucleotide sequence is likely to permit the overall homology of the rest of the sequence to be low, but not affect the dosage

It should be understood that selection of the necessary homology, selection of the defaults for the program and selection of the program employed to calculate homology is within the skill of the art, given the teachings of this specification and the knowledge extant in the scientific literature.

The RNA molecule polynucleotide sequence is also desirably substantially non-homologous to any naturally occurring, normally functioning, and essential mammalian polynucleotide sequence, so that the RNA molecule polynucleotide sequence does not adversely affect the function of any essential naturally occurring mammalian polynucleotide sequence, when used in this invention. Such naturally occurring functional mammalian polynucleotide sequences include mammalian sequences that encode desired proteins, as well as mammalian sequences that are non-coding, but that provide for essential regulatory sequences in a healthy mammal. Essentially, the RNA molecule useful in this invention must be sufficiently distinct in sequence from any mammalian polynucleotide sequence for which the function is intended to be undisturbed after any of the methods of this invention is performed. As described for determining the homology to the target sequence above, one of skill in the art may have resort to the above-identified computer algorithms to define the essential lack of homology between the RNA molecule polynucleotide sequence and the normal mammalian sequences. Thus, in one exemplary embodiment, the homology between the RNA polynucleotide and the selected normal sequence is less than the homologies of the formulae described above. More preferably, there is almost no homology at all between the RNA polynucleotide and any normal mammalian sequence. It should be understood that selection of the necessary homology is within the skill of the art, given the teachings of this specification and the knowledge extant in the scientific literature.

Finally, yet another desirable attribute of the RNA molecule of the composition for use in the present invention is that it does not produce a functional protein, or alternatively, is not translated. The RNA molecule or the delivery agent can be engineered in a variety of known ways, so as to optionally not express a functional protein or to optionally not interact with cellular factors involved in translation. Thus, for example, the agent, whether it be a synthesized RNA molecule or an agent which becomes an RNA molecule *in vivo,* lacks a poly-adenylation sequence. Similarly, the agent can lack a Kozak region necessary for protein translation. In another embodiment, the RNA molecule can also lacks the native initiating methionine codon. In still another embodiment, the RNA molecule polynucleotide sequence lacks a cap structure. In yet a further embodiment, the RNA molecule has no signals for protein synthesis. In still another embodiment, the RNA molecule contains no coding sequence or a functionally inoperative coding sequence. In still another embodiment, the RNA sequence can be punctuated with intronic sequences. In yet a further embodiment, a hairpin sequence can be placed before the native initiation codon, if present. In still another embodiment, the RNA molecule can be an RNA/DNA hybrid as described above. All such embodiments can be designed by resort to the known teachings of, e.g., such texts as cited below.

The following are various specific embodiments that may be used to achieve polynucleotide inhibition as described herein. It should be recognized that the various RNA (and RNA/DNA hybrid) structures described below may be used singly or in any combination of two or more, e.g., a lariat (sense or antisense) and/or a complementary circular and/or linear molecule. The antisense lariat or circle structures may also be used alone. Furthermore, these structures may include regions of self complementarity (e.g., tandem sense and antisense sequences) as well as additional antisense sequences relative to a desired target. Throughout this document the term "antisense" is used to mean complementary to and capable of hybridizing with any mRNA.

In one embodiment, polynucleotides in the form of "lariats" may be utilized. Lariats contain a 2'-5' phosphodiester linkage as opposed to the usual 3'-5' linkage. Such structures are formed in splicing reactions catalyzed by spliceosomes and self-cleaving ribozymes. These structures are either intermediates or by-products of splicing reactions. They can be prepared *in vivo* through expression (transcription) in a cell or prepared *in vitro.* Lariats form when a free 5' phosphoryl group of either a ribose or deoxyribose becomes linked to the 2' -OH of a ribose in a loop back fashion. The lariats may contain 10 or more nucleotides in the loop or may be a complete circle, with the loop back linkage in each case being 2'-5'. A lariat linking the terminal nucleotides produces a circle-like structure. The loops and/or the stem can contain either the sense and the antisense sequences in tandem in a single molecule, or each single lariat contains either a sense or an antisense sequence. The lariats that contain sense and antisense in separate molecules may be administered together as a double-stranded form or the antisense lariat may be used singly to form a double strand with the mRNA in the cell. Lariats may be RNA or a DNA hybrid, with the 2'-5' linkage effected through the 2' -OH of the RNA portion of the hybrid [Rees C and Song Q. Nucl. Acid Res., 27. 2672-2681 (1999); Dame E et al, Biochemistry, 38, 3157-3167, 1999; Clement J.Q. et al, RNA, 5, 206-220, 1999; Block T and Hill J. J. Neurovirol., 3, 313-321, 1997; Schindewolf CA and Domdey H., Nucl. Acid Res., 23, 1133-1139 (1995)].

In another embodiment, a circular RNA (or circular RNA-DNA hybrid) can be generated through a 2'-5' or a 3'-5' linkage of the terminii. These may be generated enzymatically through RNA ligase reactions using a splinter to bring the ends in proximity *in vitro,* or through the use of self splicing ribozymes (*in vivo* and *in vitro*). The desired inhibition may be achieved by providing one or more RNA circles, made *in vitro* or expressed *in vivo,* including single circles with or without self complementarity, as well as double stranded circular RNA (both sense and antisense strands relative to the target polynucleotide), or two circles of single-stranded RNA which have regions of complementarity to each other as well as one having complementarity to a target.

Another embodiment utilizes single RNA (or RNA-DNA hybrid) antisense circles (circular RNA without self complementarity which is complementary to the target mRNA). Still another embodiment utilizes RNA-DNA circles or a circular DNA molecule complementary to a target mRNA molecule. Single circles with tandem sense and antisense sequences (in any order) which have complementarity to a target message may be used as the composition which inhibits the function of the target sequence. It may be preferred to use circular molecules having such self-complementary sequences which may form rod-like sections, as well as additional antisense sequences to the target [Schindewolf CA and Domdey H. Nucl. Acid Res., 23, 1133-1139 (1995); Rees C and Song Q., Nucl. Acid Res., 27, 2672-2681 (1999); Block T and Hill J., J Neurovirol., 3, 313-321(1997)].

In yet a further embodiment, the composition which inhibits the target sequence is a capped linear RNA. Whether the dsRNA is formed *in vitro* or *in vivo,* either one or both strands may be capped. In circumstances where cytoplasmic expression would not ordinarily result in capping of the RNA molecule, capping may be accomplished by various means including use of a capping enzyme, such as a vaccinia capping enzyme or an alphavirus capping enzyme. A capped antisense molecule may be used to achieve the desired post transcriptional silencing of the target gene. Capped RNA may be prepared *in vitro* or *in vivo.* RNA made in the nucleus by RNA polII ordinarily is capped. Cytoplasmically expressed RNA may or may not be capped. Capping can be achieved by expressing capping enzymes of cytoplasmic viruses. Either both capped or one capped and one uncapped or both uncapped RNA or RNA-DNA hybrid sequences may be used in these compositions. Capped or uncapped antisense molecule may be used, singly or in any combination with polynucleotide structures described herein.

The RNA molecule for use in this invention may be delivered to the mammalian cell in the composition as an RNA molecule or partially double stranded RNA sequence, or RNA/DNA hybrid, which was made *in vitro* by conventional enzymatic synthetic methods using, for example, the bacteriophage T7, T3 or SP6 RNA polymerases according to the conventional methods described by such texts as the Promega Protocols and Applications Guide, (3rd ed. 1996), eds. Doyle, ISBN No. 1-882274-57-1.

Alternatively these molecules may be made by chemical synthetic methods *in vitro* [see, e.g., Q. Xu et al, Nucl. Acids Res., 24(18):3643-4 (Sept. 1996); N. Naryshkin et al, Bioorg. Khim., 22(9):691-8 (Sept. 1996); J. A. Grasby et al, Nucl. Acids Res., 21(19):4444-50 (Sept 1993); C. Chaix et al, Nucl, Acids Res., 17(18):7381-93 (1989); S.H. Chou et al, Biochem., 28(6):2422-35 (Mar. 1989); O. Odai et al, Nucl Acids Symp. Ser., 21:105-6 (1989); N.A. Naryshkin et al, Bioorg. Khim, 22(9):691-8 (Sept. 1996); S. Sun et al, RNA, 3(11):1352-1363 (Nov. 1997); X. Zhang et al, Nucl Acids Res., 25(20):3980-3 (Oct. 1997); S. M. Grvaznov et al, Nucl, Acids Res., 26(18):4160-7 (Sept. 1998); M. Kadokura et al, Nucl. Acids Symp Ser, 37:77-8 (1997); A. Davison et al, Biomed. Pept. Proteins. Nucl. Acids, 2(1):1-6 (1996); and A. V. Mudrakovskaia et al, Bioorg. Khim., 17(6):819-22 (Jun. 1991)].

Still alternatively, the RNA molecule for use in this invention can be made in a recombinant microorganism, e.g., bacteria and yeast or in a recombinant host cell, e.g., mammalian cells, and isolated from the cultures thereof by conventional techniques. See, e.g., the techniques described in Sambrook et al, MOLECULAR CLONING, A LABORATORY MANUAL, 2nd Ed.; Cold Spring Harbor Laboratory Press, Cold Spring Harbor, New York, 1989, which is exemplary of laboratory manuals that detail these techniques, and the techniques described in US Patent Nos. 5,824,538; 5,877,159 and 65,643,771.

Such RNA molecules prepared or synthesized *in vitro* may be directly delivered to the mammalian cell or to the mammal as they are made *in vitro.* The references above provide one of skill in the art with the techniques necessary to produce any of the following specific embodiments, given the teachings provided herein. Therefore, in one embodiment, the "agent" of the composition is a duplex (i.e., it is made up of two strands), either complete or partially double stranded RNA. In another embodiment, the agent is a single stranded RNA sense strand. In another embodiment, the agent of the composition is a single stranded RNA anti-sense strand. Preferably the single stranded RNA sense or anti-sense strand forms a hairpin at one or both termini. Desirably, the single stranded RNA sense or anti-sense strand forms a hairpin at some intermediate portion between the termini. Such a single stranded RNA sense or anti-sense strand may also be designed to fold back upon itself to become partially double stranded *in vitro* or *in vivo.* Yet another embodiment of an extant RNA molecule as the effective agent used in the compositions is a single stranded RNA sequence comprising both a sense polynucleotide sequence and an anti-sense polynucleotide sequence, optionally separated by a non-base paired polynucleotide sequence. Preferably, this single stranded RNA sequence has the ability to become double-stranded once it is in the cell, or *in vitro* during its synthesis. Still another embodiment of this invention is an RNA/DNA hybrid as described above. Still another embodiment of the synthetic RNA molecule is a circular RNA molecule that optionally forms a rod structure [see, e.g., K-S. Wang et al, Nature, 323:508-514 (1986)] or is partially double-stranded, and can be prepared according to the techniques described in S. Wang et al, Nucl. Acids Res., 22(12):2326-33 (June 1994); Y. Matsumoto et al, Proc. Natl. Acad. Sci., USA. 87(19)7628-32 (Oct. 1990); Proc. Natl. Acad. Sci., USA, 91(8):3117-21 (Apr. 1994); M. Tsagris et al, Nucl. Acids Res., 19(7):1605-12 (Apr. 1991); S. Braun et al, Nucl. Acids Res., 24(21):4152-7 (Nov. 1996); Z. Pasman et al, RNA, 2(6):603-10 (Jun. 1996); P. G. Zaphiropoulos, Proc. Natl. Acad. Sci., USA, 93(13):6536-41 (Jun. 1996); D. Beaudry et al, Nucl. Acids Res., 23(15):3064-6 (Aug. 1995). Still another agent is a double-stranded molecule comprised of RNA and DNA present on separate strands, or interspersed on the same strand.

Alternatively, the RNA molecule may be formed *in vivo* and thus delivered by a "delivery agent" which generates such a partially double-stranded RNA molecule *in vivo* after delivery of the agent to the mammalian cell or to the mammal. Thus, the agent which forms the composition for use in this invention is, in one embodiment, a double stranded DNA molecule "encoding" one of the above-described RNA molecules. The DNA agent provides the nucleotide sequence which is transcribed within the cell to become a double stranded RNA. In another embodiment, the DNA sequence provides a deoxyribonucleotide sequence which within the cell is transcribed into the above-described single stranded RNA sense or anti-sense strand, which optionally forms a hairpin at one or both termini or folds back upon itself to become partially double stranded. The DNA molecule which is the delivery agent of the composition can provide a single stranded RNA sequence comprising both a sense polynucleotide sequence and an anti-sense polynucleotide sequence, optionally separated by a non-base paired polynucleotide sequence, and wherein the single stranded RNA sequence has the ability to become double-stranded. Alternatively, the DNA molecule which is the delivery agent provides for the transcription of the above-described circular RNA molecule that optionally forms a rod structure or partial double strand *in vivo.* The DNA molecule may also provide for the in vivo production of an RNA/DNA hybrid as described above, or a duplex containing one RNA strand and one DNA strand. These various DNA molecules may be designed by resort to conventional techniques such as those described in Sambrook, cited above or in the Promega reference, cited above.

A latter delivery agent for use in the present invention, which enables the formation in the mammalian cell of any of the above-described RNA molecules, can be a DNA single stranded or double stranded plasmid or vector. Expression vectors designed to produce RNAs as described herein *in vitro* or *in vivo* may containing sequences under the control of any RNA polymerase, including mitochondrial RNA polymerase, RNA poll, RNA polII, and RNA polIII. These vectors can be used to transcribe the desired RNA molecule in the cell according to this invention. Vectors may be desirably designed to utilize an endogenous mitochondrial RNA polymerase (e.g., human mitochondrial RNA polymerase, in which case such vectors may utilize the corresponding human mitochondrial promoter). Mitochondrial polymerases may be used to generate capped (through expression of a capping enzyme) or uncapped messages *in vivo.* RNA pol I, RNA pol II, and RNA pol III transcripts may also be generated *in vivo.* Such RNAs may be capped or not, and if desired, cytoplasmic capping may be accomplished by various means including use of a capping enzyme such as a vaccinia capping enzyme or an alphavirus capping enzyme. The DNA vector is designed to contain one of the promoters or multiple promoters in combination (mitochondrial, RNA poll, II, or polIII, or viral, bacterial or bacteriophage promoters along with the cognate polymerases). Preferably, where the promoter is RNA pol II, the sequence encoding the RNA molecule has an open reading frame greater than about 300 nts to avoid degradation in the nucleus. Such plasmids or vectors can include plasmid sequences from bacteria, viruses or phages. Such vectors include chromosomal, episomal and virus-derived vectors e.g., vectors derived from bacterial plasmids, bacteriophages, yeast episomes, yeast chromosomal elements, and viruses, vectors derived from combinations thereof, such as those derived from plasmid and bacteriophage genetic elements, cosmids and phagemids. Thus, one exemplary vector is a single or double-stranded phage vector. Another exemplary vector is a single or double-stranded RNA or DNA viral vector. Such vectors may be introduced into cells as polynucleotides, preferably DNA, by well known techniques for introducing DNA and RNA into cells. The vectors, in the case of phage and viral vectors may also be and preferably are introduced into cells as packaged or encapsidated virus by well known techniques for infection and transduction. Viral vectors may be replication competent or replication defective. In the latter case, viral propagation generally occurs only in complementing host cells.

In another embodiment the delivery agent comprises more than a single DNA or RNA plasmid or vector. As one example, a first DNA plasmid can provide a single stranded RNA sense polynucleotide sequence as described above, and a second DNA plasmid can provide a single stranded RNA anti-sense polynucleotide sequence as described above, wherein the sense and anti-sense RNA sequences have the ability to base-pair and become double-stranded. Such plasmid(s) can comprise other conventional plasmid sequences, e.g., bacterial sequences such as the well-known sequences used to construct plasmids and vectors for recombinant expression of a protein. However, it is desirable that the sequences which enable protein expression, e.g., Kozak regions, etc., are not included in these plasmid structures.

The vectors designed to produce dsRNAs for use in the invention may desirably be designed to generate two or more, including a number of different dsRNAs homologous and complementary to a target sequence. This approach is desirable in that a single vector may produce many, independently operative dsRNAs rather than a single dsRNA molecule from a single transcription unit and by producing a multiplicity of different dsRNAs, it is possible to self select for optimum effectiveness. Various means may be employed to achieve this, including autocatalytic sequences as well as sequences for cleavage to create random and/or predetermined splice sites.

Other delivery agents for providing the information necessary for formation of the above-described desired RNA molecules in the mammalian cell include live, attenuated or killed, inactivated recombinant bacteria which are designed to contain the sequences necessary for the required RNA molecules of this invention. Such recombinant bacterial cells, fungal cells and the like can be prepared by using conventional techniques such as described in US Patent Nos. 5,824,538; 5,877,159 and 65,643,771. Microorganisms useful in preparing these delivery agents include those listed in the above cited reference, including, without limitation, *Escherichia coli, Bacillus subtilis, Salmonella typhimurium,* and various species of *Pseudomonas, Streptomyces,* and *Staphylococcus.*

Still other delivery agents for providing the information necessary for formation of the desired, above-described RNA molecules in the mammalian cell include live, attenuated or killed, inactivated viruses, and particularly recombinant viruses carrying the required RNA polynucleotide sequence discussed above. Such viruses may be designed similarly to recombinant viruses presently used to deliver genes to cells for gene therapy and the like, but preferably do not have the ability to express a protein or functional fragment of a protein. Among useful viruses or viral sequences which may be manipulated to provide the required RNA molecule to the mammalian cell *in vivo* are, without limitation, alphavirus, adenovirus, adeno-associated virus, baculoviruses, delta virus, pox viruses, hepatitis viruses, herpes viruses, papova viruses (such as SV40), poliovirus, pseudorabies viruses, retroviruses, vaccinia viruses, positive and negative stranded RNA viruses, viroids, and virusoids, or portions thereof. These various viral delivery agents may be designed by applying conventional techniques such as described in M. Di Nocola et al, Cancer Gene Ther., 5(6):350-6 (1998), among others, with the teachings of the present invention.

Another delivery agent for providing the information necessary for formation of the desired, above-described RNA molecules in the mammalian cell include live, attenuated or killed, inactivated donor cells which have been transfected or infected *in vitro* with a synthetic RNA molecule or a DNA delivery molecule or a delivery recombinant virus as described above. These donor cells may then be administered to the mammal, as described in detail below, to stimulate the mechanism in the mammal which mediates this inhibitory effect. These donor cells are desirably mammalian cells, such as C127, 3T3, CHO, HeLa, human kidney 293, BHK cell lines, and COS-7 cells, and preferably are of the same mammalian species as the mammalian recipient. Such donor cells can be made using techniques similar to those described in, e.g., Emerich et al, J. Neurosci., 16: 5168-81 (1996). Even more preferred, the donor cells may be harvested from the specific mammal to be treated and made into donor cells by *ex vivo* manipulation, akin to adoptive transfer techniques, such as those described in D. B. Kohn et al, Nature Med., 4(7):775-80 (1998). Donor cells may also be from non-mammalian species, if desired.

Finally, the composition for use in this invention can also include one or more of the selected agents which are described above. The composition can contain a mixture of synthetic RNA molecules described above, synthetic DNA delivery molecules described above, and any of the other delivery agents described above, such as recombinant bacteria, cells, and viruses. The identity of the composition mixture may be readily selected by one of skill in the art.

### B. Pharmaceutical (Therapeutic or Prophylactic) Compositions of the Invention

The compositions of this invention for pharmaceutical use desirably contain the synthetic RNA molecule as described above or the agent which provides that RNA molecule to the mammalian cell *in vivo* in a pharmaceutically acceptable carrier, with additional optional components for pharmaceutical delivery. The specific formulation of the pharmaceutical composition depends upon the form of the agent delivering the RNA molecule.

Suitable pharmaceutically acceptable carriers facilitate administration of the polynucleotide compositions of this invention, but are physiologically inert and/or nonharmful. Carriers may be selected by one of skill in the art. Such carriers include but are not limited to, sterile saline, phosphate, buffered saline, dextrose, sterilized water, glycerol, ethanol, lactose, sucrose, calcium phosphate, gelatin, dextran, agar, pectin, peanut oil, olive oil, sesame oil, and water and combinations thereof. Additionally, the carrier or diluent may include a time delay material, such as glycerol monostearate or glycerol distearate alone or with a wax. In addition, slow release polymer formulations can be used. The formulation should suit not only the form of the delivery agent, but also the mode of administration. Selection of an appropriate carrier in accordance with the mode of administration is routinely performed by those skilled in the art.

Where the composition contains the synthetic RNA molecule or where the agent is another polynucleotide, such as, a DNA molecule, plasmid, viral vector, or recombinant virus, or multiple copies of the polynucleotide or different polynucleotides, etc., as described above, the composition may desirably be formulated as "naked" polynucleotide with only a carrier. Alternatively, such compositions desirably contain optional polynucleotide facilitating agents or "co-agents", such as a local anaesthetic, a peptide, a lipid including cationic lipids, a liposome or lipidic particle, a polycation such as polylysine, a branched, three-dimensional polycation such as a dendrimer, a carbohydrate, a cationic amphiphile, a detergent, a benzylammonium surfactant, or another compound that facilitates polynucleotide transfer to cells. Non-exclusive examples of such facilitating agents or co-agents useful in this invention are described in U. S. Patent Nos. 5,593,972; 5,703,055; 5,739,118; 5,837,533 and International Patent Application No. WO96/10038, published April 4, 1996; and International Patent Application No WO94/16737, published August 8, 1994.

When the facilitating agent used is a local anesthetic, preferably bupivacaine, an amount of from about 0.1 weight percent to about 1.0 weight percent based on the total weight of the polynucleotide composition is preferred. See, also, International Patent Application No. PCT/US98/22841, which teaches the incorporation of benzylammonium surfactants as co-agents, administered in an amount of between about 0.001-0.03 weight %.

Where the delivery agent of the composition is other than a polynucleotide composition, e.g., is a transfected donor cell or a bacterium as described above, the composition may also contain other additional agents, such as those discussed in US Patents No. 5,824,538; 5,643,771; 5,877,159.

Still additional components that may be present in any of the compositions are, adjuvants, preservatives, chemical stabilizers, or other antigenic proteins. Typically, stabilizers, adjuvants, and preservatives are optimized to determine the best formulation for efficacy in the target human or animal. Suitable exemplary preservatives include chlorobutanol, potassium sorbate, sorbic acid, sulfur dioxide, propyl gallate, the parabens, ethyl vanillin, glycerin, phenol, and parachlorophenol. Suitable stabilizing ingredients which may be used include, for example, casamino acids, sucrose, gelatin, phenol red, N-Z amine, monopotassium diphosphate, lactose, lactalbumin hydrolysate, and dried milk. A conventional adjuvant is used to attract leukocytes or enhance an immune response. Such adjuvants include, among others, Ribi, mineral oil and water, aluminum hydroxide, Amphigen, Avridine, L121/squalene, D-lactide-polylactide/glycoside, pluronic plyois, muramyl dipeptide, killed *Bordetella,* and saponins, such as Quil A.

In addition, other agents which may function as transfecting agents and/or replicating agents and/or inflammatory agents and which may be co-administered with the composition include growth factors, cytokines and lymphokines such as alpha-interferon, gamma-interferon, platelet derived growth factor (PDGF), colony stimulating factors, such as G-CSF, GM-CSF, tumor necrosis factor (TNF), epidermal growth factor (EGF), and the interleukins, such as IL-1, IL-2, IL-4, IL-6, IL-8, IL-10 and IL-12. Further, fibroblast growth factor, surface active agents such as immune-stimulating complexes (ISCOMS), Freund's incomplete adjuvant, LPS analog including monophosphoryl Lipid A (MPL), muramyl peptides, quinone analogs and vesicular complexes such as squalene and squalene, and hyaluronic acid may also be used administered in conjunction with the compositions.

The pharmaceutical compositions may also contain other additives suitable for the selected mode of administration of the composition. Thus, these compositions can contain additives suitable for administration via any conventional route of administration, including without limitation, parenteral administration, intraperitoneal administration, intravenous administration, intramuscular administration, subcutaneous administration, intradermal administration, oral administration, topical administration, intranasal administration, intra-pulmonary administration, rectal administration, vaginal administration, and the like. All such routes are suitable for administration of these compositions, and may be selected depending on the agent used, patient and condition treated, and similar factors by an attending physician.

The composition for use of the invention may also involve lyophilized polynucleotides, which can be used with other pharmaceutically acceptable excipients for developing powder, liquid or suspension dosage forms, including those for intranasal or pulmonary applications. See, e.g., Remington: The Science and Practice of Pharmacy, Vol. 2, 19th edition (1995), e.g., Chapter 95 Aerosols; and International Patent Application No. PCT/US99/05547. Routes of administration for these compositions may be combined, if desired, or adjusted.

In some preferred embodiments, the pharmaceutical compositions for use of the invention are prepared for administration to mammalian subjects in the form of, for example, liquids, powders, aerosols, tablets, capsules, enteric coated tablets or capsules, or suppositories.

The compositions for use of the present invention, when used as pharmaceutical compositions, can comprise about 1 ng to about 20 mgs of polynucleotide molecules as the delivery agent of the compositions, e.g., the synthetic RNA molecules or the delivery agents which can be DNA molecules, plasmids, viral vectors, recombinant viruses, and mixtures thereof. In some preferred embodiments, the compositions contain about 10 ng to about 10 mgs of polynucleotide sequences. In other embodiments, the pharmaceutical compositions contain about 0.1 to about 500 µg polynucleotide sequences. In some preferred embodiments, the compositions contain about 1 to about 350 µg polynucleotide sequences. In still other preferred embodiments, the pharmaceutical compositions contain about 25 to about 250 µg of the polynucleotide sequences. In some preferred embodiments, the vaccines and therapeutics contain about 100 µg of the polynucleotide sequences.

The compositions for use of the present invention in which the delivery agents are donor cells or bacterium can be delivered in dosages of between about 1 cell to about 10⁷ cells/dose. Similarly, where the delivery agent is a live recombinant virus, a suitable vector-based composition contains between 1x10² pfu to 1x10¹² pfu per dose.

Given the teachings of this invention, and the observed capacity of the inhibitory effect of the methods and compositions to be propagated to more cells than the cells transfected or infected with the composition for use of this invention, it is likely that suitable dosage adjustments can be made downwards from the above-noted dosages. Thus, the above dosage ranges are guidelines only. In general, the pharmaceutical compositions are administered in an amount effective to inhibit or reduce the function of the target polynucleotide sequence for treatment or prophylaxis of the diseases, disorders or infections for which such target functions are necessary for further propagation of the disease or causative agent of the disease. The amount of the pharmaceutical composition in a dosage unit employed is determined empirically, based on the response of cells *in vitro* and response of experimental animals to the compositions. Optimum dosage is determined by standard methods for each treatment modality and indication. Thus the dose, timing, route of administration, and need for readministration of these compositions may be determined by one of skill in the art, taking into account the condition being treated, its severity, complicating conditions, and such factors as the age, and physical condition of the mammalian subject, the employment of other active compounds, and the like.

### C. Therapeutic and Prophylactic Uses of the Invention

The composition for use in a method of this invention can employ the compositions described in detail above, and possibly other polynucleotide sequences currently used in the art (e.g., polynucleotide molecules which do encode proteins, whether functional or non-functional, or known RNA catalytic sequences, such as ribozymes) which can provide partially double stranded RNA molecules to a mammalian cell. It is anticipated, however, that the efficiency of these methods is enhanced by the use of RNA molecules which do not produce protein. These methods reduce or inhibit the function of a target polynucleotide sequence(s) in a mammal or in the cell of a mammal. The compositions, pharmaceutical compositions, dosages and modes of administration described above are particularly desirable for the treatment of a variety of disorders that plague mammals, including infections by heterologous intracellular pathogenic organisms. Additionally, the compositions for use of this invention are useful in preventing infection of a mammal with a pathogen, or preventing the occurrence of disorders caused by reactivation of a latent pathogen. These compositions are also useful for the treatment of pathogenically-induced cancers.

One embodiment of a method of treatment is a method for treating a viral infection in a mammal. Particularly suitable for such treatment area DNA viruses or viruses that have an intermediary DNA stages. Among such viruses are included, without limitation, viruses of the species Retrovirus, Herpesvirus, Hepadenovirus, Poxvirus, Parvovirus, Papillomavirus, and Papovavirus. Specifically some of the more desirable viruses to treat with this method include, without limitation, HIV, HBV, HSV, CMV, HPV, HTLV and EBV. The agent used in this method provides to the cell of the mammal an at least partially double stranded RNA molecule as described above, which is substantially homologous to a target polynucleotide which is a virus polynucleotide sequence necessary for replication and/or pathogenesis of the virus in an infected mammalian cell. Among such target polynucleotide sequences are non-coding viral polynucleotide sequences which provide regulatory functions necessary for transfer of the infection from cell to cell, e.g., the HIV LTR, and other viral promoter sequences, such as HSV vp16 promoter, HSV-ICP0 promoter, HSV- ICP4, ICP6 and gD promoters, the HBV surface antigen promoter, the HBV pre-genomic promoter, among others. As described above, the composition is administered with an polynucleotide uptake enhancer or facilitator and an optional pharmaceutically acceptable carrier. The amount or dosage which is administered to the mammal is effective to reduce or inhibit the function of the viral sequence in the cells of the mammal.

While not wishing to be bound by theory, once the RNA molecule is delivered to or produced in a cell infected by the virus, the exogenous RNA molecule reduces or inhibits (i.e. turns off) the homologous viral sequence and is itself inhibited, so that the function of the viral sequence is reduced or inhibited. As demonstrated in the examples below, the inhibition of function effect is transferred from the mammalian cell which receives the exogenous RNA molecule to other mammalian cells in the subject which have not directly been provided with the exogenous RNA molecule. It is presently theorized that this results occurs on the level of RNA degradation.

Thus, this method can be used to treat mammalian subjects already infected with a virus, such as HIV, in order to shut down or inhibit a viral gene function essential to virus replication and/or pathogenesis, such as HIV gag. Alternatively, this method can be employed to inhibit the functions of viruses which exist in mammals as latent viruses, e.g., *Varicella zoster* virus, and are the causative agents of the disease known as shingles. Similarly, diseases such as atherosclerosis, ulcers, chronic fatigue syndrome, and autoimmune disorders, recurrences of HSV-1 and HSV-2, HPV persistent infection, e.g., genital warts, and chronic HBV infection among others, which have been shown to be caused, at least in part, by viruses, bacteria or another pathogen, can be treated according to this method by targeting certain viral polynucleotide sequences essential to viral replication and/or pathogenesis in the mammalian subject.

In still another embodiment of this invention, the compositions described above can be employed in a method to prevent viral infection in a mammal. When the method described above, i.e., administering a composition described above in an amount effective to reduce or inhibit the function of the essential target viral polynucleotide sequence to a mammal, is administered prior to exposure of the mammal to the virus, it is expected that the exogenous RNA molecule remains in the mammal and work to inhibit any homologous viral sequence which presents itself to the mammal thereafter. Thus, the compositions may be used to inhibit or reduce the function of a viral polynucleotide sequence for vaccine use.

Still an analogous embodiment of the above "anti-viral" methods of the invention includes a method for treatment or prophylaxis of a virally induced cancer in a mammal. Such cancers include HPV E6/E7 virus-induced cervical carcinoma, HTLV-induced cancer, and EBV induced cancers, such as Burkitts lymphoma, among others. This method is accomplished by administering to the mammal a composition as described above in which the target polynucleotide is a a non-expressed regulatory sequence, which sequence function is required for the maintenance of the tumor in the mammal. Among such sequences are included, without limitation, HPV16 E6 and E7 sequences and HPV 18 E6 and E7 sequences. Others may readily be selected by one of skill in the art. The composition is administered in an amount effective to reduce or inhibit the function of the antigen in the mammal, and preferably employs the composition components, dosages and routes of administration as described above. The molecular mechanism underlying this method is the same as that described above.

In another embodiment of the invention, the compositions for use of this invention can be employed in a method for the treatment or prophylaxis of infection of a mammal by a non-viral intracellular pathogen. As used herein, the term "intracellular pathogen" is meant to refer to a virus, bacteria, protozoan or other pathogenic organism that, for at least part of its reproductive or life cycle, exists within a host cell and therein produces or causes to be produced, pathogenic proteins. Intracellular pathogens which infect cells which include a stage in the life cycle where they are intracellular pathogens include, without limitation, Listeria, Chlamydia, Leishmania, Brucella, Mycobacteria, Shigella, and as well as Plasmodia, e.g., the causative agent of malaria, *P. falciparum.* Extracellular pathogens are those which replicate and/or propagate outside of the mammalian cell, e.g., Gonorrhoeae, and Borrellia, among others. According to this embodiment, such infection by an pathogen may be treated or possibly prevented by administering to a mammalian subject, either already infected or anticipating exposure to the pathogen, with a composition as described above with an optional second agent that facilitates polynucleotide uptake in a cell, in a pharmaceutically acceptable carrier. In this case, the RNA molecule of the composition has a polynucleotide sequence which is substantially homologous to a target polynucleotide sequence of the pathogen that is necessary for replication and/or pathogenesis of the pathogen in an infected mammal or mammalian cell. As above, the amount of the composition administered is an amount effective to reduce or inhibit the function of the pathogenic sequence in the mammal. The dosages, timing, routes of administration and the like are as described above.

One of skill in the art, given this disclosure can readily select viral families and genera, or pathogens including prokaryotic and eukaryotic protozoan pathogens as well as multicellular parasites, for which therapeutic or prophylactic compositions for use according to the present invention can be made. See, e.g., the tables of such pathogens in general immunology texts and in U. S. Patent No. 5,593,972.

The compositions for use of this invention and possibly protein-encoding molecules of the prior art may also be employed in another novel method. Such compositions are also useful in the treatment of certain non-pathogenic diseases or disorders of mammals, such as certain cancers or inherited disorders. Among conditions particularly susceptible to treatment or prophylaxis according to this invention are those conditions which are characterized by the presence of an aberrant mammalian polynucleotide sequence, the function of which is necessary to the initiation or progression of the disorder, but can be inhibited without causing harm or otherwise unduly adversely impacting the health of the mammal. In other words, a characteristic of a disorder suitable for this treatment is that the mammal can survive without the function of the gene, or can survive if the function of the gene was substantially reduced. In such cases, the function of the aberrant or abnormal polynucleotide sequence can be replaced exogenously by therapy. In another case, the disease can be caused by the presence or function of an abnormal polynucleotide sequence or gene in a mammal, where the mammal also possesses a normal copy of the polynucleotide sequence or gene, and wherein the differences between the abnormal gene and the normal gene are differences in nucleotide sequence. In such cases, inhibition of the function of the abnormal polynucleotide sequence by the method described herein is likely to permit the normal polynucleotide sequence to function, without exogenous treatment.

Thus, in one embodiment, a method of treatment or prophylaxis of a cancer in a mammal involves administering to the mammal a composition for use of this invention in which the target polynucleotide sequence is an abnormal cancer-causing polynucleotide sequence or gene in a mammal. The composition for use of this invention is administered in an amount effective to reduce or inhibit the function of the abnormal sequence in the mammal. As described above, the composition can contain an optional second agent that facilitates polynucleotide uptake in a cell, and a pharmaceutically acceptable carrier, and be administered in dosages, regimens and by routes as described above.

Mammalian cancers which are characterized by the presence of abnormal and normal polynucleotide sequences include chronic myelogenous leukemia (CML) and acute lymphoblastic leukemia (ALL), where the abnormal sequence is a fusion of two normal genes, i.e., *bcr-abl.* See, e.g., the description of these cancers in International Patent Publication No. WO94/13793, published June 23, 1994. In such cancers or diseases, such as CML, the afflicted mammal also possesses a normal copy of the polynucleotide sequence or gene, and the differences between the abnormal and normal sequences or genes are differences in nucleotide sequence. For example, for CML, the abnormal sequence is the bcr-abl fusion, while the normal sequence is bcr and abl. Thus, the method above can be employed with the target polynucleotide sequence being the sequence which spans the fusion. A method of treatment or prophylaxis of such a cancer in a mammal comprises administering to the mammal a composition for use of this invention wherein the target polynucleotide is a polynucleotide sequence of an abnormal cancer-causing gene in a mammal which also possesses a normal copy of the gene, and wherein the differences between the abnormal gene and the normal gene are differences in polynucleotide sequence. The composition is administered as above, with an optional second agent that facilitates polynucleotide uptake in a cell, and in a pharmaceutically acceptable carrier and in an amount effective to reduce or inhibit the function of the abnormal sequence in the mammal.

The present invention thus encompasses methods for evoking the above-described molecular mechanism for treating any disease or disorder in a mammal characterized by expression of an undesirable polynucleotide product or polynucleotide mediated function not found in a healthy mammal by use of a composition which can deliver to the cells of the mammal the partially double-stranded RNA molecule substantially homologous to the target polynucleotide sequence which expresses or mediates the undesired product or function, in an amount effective to reduce or inhibit the function of that polynucleotide in the cells of the mammal. Provided that the RNA molecule is sufficiently non-homologous to essential mammalian polynucleotide sequences, so that it does not inhibit the function of those essential sequences, this method can be clearly seen to have many therapeutic and prophylactic uses. One of skill in the art can readily select disorders described above, and can also readily select target polynucleotide sequences against which the compositions of the present invention are directed.

### D. Other Methods

The compositions described above, and the general methods of using these compositions to inhibit or reduce the function of a target polynucleotide sequence, can also be applied to a variety of research, and *in vitro* applications. For example, they can be applied to research to determine the function of a selected polynucleotide sequence in a cell line, or a mammalian laboratory animal, by administering to that cell in tissue culture or that animal *in vivo* a composition described herein wherein the RNA molecule polynucleotide sequence is substantially homologous to the selected sequence and preferably substantially non-homologous to other polynucleotide sequences in the animal. The inhibition of the function of that target sequence permits study of its influence on the animal's biology and physiology.

Similarly, application of this method can be used to make cell lines of mammalian, bacterial, yeast, fungal, insect and other origins defective in selected pathways by "silencing" a selected functional sequence, such as an enzymatic sequence, a protein expressing sequence, or regulatory sequences necessary to the expression thereof. Such manipulated cells may be employed in conventional assays or drug screening assays, etc.

In an analogous method, a "knock-out" laboratory animal can be prepared by altering the dosage of administration sufficient to permanently shut off the function of a selected gene. Thus, the method in delivering an RNA molecule with a polynucleotide sequence sufficiently homologous to the sequence selected to be "knocked out" in the laboratory animal as described above provides a simpler technique for developing "knock-out" mice and other laboratory animals useful for pharmaceutical and genetic research.

Still other research methods for use of the compositions include the preparation of mutants of microorganisms, both eukaryotic and prokaryotic, for use as research agents or as industrial production strains for the microbial production of desired proteins. Still other uses are expected to be obvious to the person of skill in the art given the teachings herein.

The following examples are illustrative only and do not limit the scope of the invention.

### EXAMPLE 1: REDUCING OR INHIBITING THE FUNCTION OF HIV p24 IN VIRALLY INFECTED CELLS

During the course of HIV infection, the viral genome is reverse transcribed into a DNA template which is integrated into the host chromosome of infected dividing cells. The integrated copy is now a blueprint from which more HIV particles are made. According to this invention, if the function of a polynucleotide sequence essential to replication and/or pathogenesis of HIV is reduced or inhibited, the viral infection can be treated. This example demonstrates the performance of one embodiment of the method of this invention.

The plasmid, HIVgpt (AIDS Research and Reference Reagent Program Catalog) was used to generate stable integrated Rhabdomyosarcoma (RD) or COS7 cell lines that contain integrated copies of the defective HIV genome, HIVgpt. The HIVgpt genome encodes a mycophenolic acid (MPA) resistance gene in place of the envelope gene and thereby confers resistance to MPA. The cell lines were made by transfecting cells with the plasmid followed by selection of cells in MPA. Cells resistant to MPA were clonally amplified. The media from the cultured clonally expanded cells was then assayed for the presence of p24 (an HIV gag polypeptide which is secreted extracellularly) using the p24 ELISA assay kit (Coulter Corporation). All cells were positive for p24.

Two RD cell lines and two COS7 cell lines are used to demonstrate one embodiment of the method of the present invention, i.e., reducing or inhibiting the function of the HIV p24 target polynucleotide, which controls p24 synthesis in these cells.

To generate a reagent of the present invention, a 600 polynucleotide (nt) sense RNA, a 600 nt antisense RNA, and a 600 bp double stranded RNA (dsRNA) mapping to the same coordinates of the gag gene of HIV strain HXB2 and lacking a cap, a poly-adenylation sequence, and a native initiation codon, are used to transfect cells. The RNA molecules are generated through transcription of PCR products bearing a bacteriophage T7 polymerase promoter at one end (see Figs. 1A and IB). The coordinates of the primers were derived from the map of the complete genome of HIV(HXB2), Genbank Accession number K03455 [see also, L. Ratner et al., AIDS Res. Hum. Retroviruses, 3(1):57-69 (1987)]. The Forward gag primer maps to coordinates 901-924 and this sequence follows the T7 promoter in the T7 Forward gag primer. The Reverse gag primer maps to coordinates 1476-1500 and follows the T7 promoter in the T7 Reverse gag primer.

To generate a composition of this invention where the agent is single-stranded sense RNA, a T7 promoter is located at the 5' end of the forward PCR primer. The PCR primers used to generate the DNA template that encodes the ss sense RNA, written 5' to 3' with the top strand of the T7 promoter underlined, are the T7 forward gag primer [SEQ ID NO: 1]:
5'GTAATACGACTCACTATAGGGCGGCAGGGAGCTAGAACGATTCGCAG 3' and the Reverse gag primer [SEQ ID NO: 2]:
5'CTGCTATGTCACTTCCCCTTGGTTC 3'

To generate a composition where the agent is a single stranded anti-sense RNA molecule, the T7 promoter is located at the 5' end of the reverse PCR primer. These primers are the T7 Reverse gag primer [SEQ ID NO: 3]:
5' GTAATACGACTCACTATAGGGCGCTGCTATGTCACTTCCCCTTGGTTC 3' and the Forward gag primer [SEQ ID NO: 4]:
5' GCAGGGAGCTAGAACGATTCGCAG 3'.

Both types of PCR products described above are included in the T7 transcription reaction to generate a composition where the agent is double-stranded RNA molecule. Alternatively, an agent of the composition according to this invention is prepared by mixing together sense and anti-sense RNA after transcription.

As a control, similarly sized sense RNA, antisense RNA, and dsRNA molecules are derived from the gD gene of a Herpes Simplex Virus, type 2 genome are generated by the same PCR and T7 transcription techniques. The coordinates of the PCR primers for HSV gD are derived from the map of GenBank Accession number K01408, HSVgD2 gene. The Forward gD primer maps to coordinates 313-336; this sequence follows the T7 promoter in the T7 Forward gD primer. The Reverse gD primer maps to coordinates 849-872, and follows the T7 promoter in the T7 Reverse gD primer. The primer sets used to generate these control molecules were:
T7 forward gD primer [SEQ ID NO: 5]:
5' GTAATACGACTCACTATAGGGCGGTCGCGGTGGGACTCCGCGTCGTC 3' and
Forward gD primer [SEQ ID NO: 6]: 5' GTCGCGGTGGGACTCCGCGTCGTC 3'; and
T7 reverse gD primer [SEQ ID NO: 7]:
5'GTAATACGACTCACTATAGGGCGGTGATCTCCGTCCAGTCGTTTATC 3' and
Reverse gD primer [SEQ ID NO: 8]: 5' GTGATCTCCGTCCAGTCGTTTATC 3'.

These RNA molecules of the invention and the above-described control molecules are assayed with the RD and COS7 cell lines as follows:
Between 5-6 x10⁵ cells/well in six-well plates are cultured to about 80-90% confluence, and are transfected with 2-3 µg of a selected RNA molecule or control molecule, using 10 µl lipofectamine (Gibco-BRL) as a transfecting agent. Transfected cells are incubated for times ranging between 1 to 17 hours. Another cell culture was transfected with doses of RNA ranging between 1 µg to 500 µgs, delivered with no known transfecting agent and incubated on the cells from 0.5 minutes to about two days. For example, one group of cells is transfected with the sense gag RNA, another with the antisense gag RNA, another with ds gag RNA, another with sense gD RNA control, another with antisense gD RNA control, and another with ds gD RNA control. Also additional negative controls are cells which receive no RNA molecules.

The cells are cultured at 37°C and monitored for p24 synthesis over the course of several weeks. The cells are assayed three times per week after two days post-administration of RNA, both by measuring p24 in the media of cells using the p24 ELISA assay kit (Coulter Corp) and by immunostaining fixed cells for p24 using a rabbit polyclonal anti-p24 sera (Intracell Corp.) and anti-rabbit IgG that is FITC-conjugated (Sigma).

According to the present invention, none of the gD RNA molecules demonstrate the ability to retard or inhibit p24 synthesis. However, according to the invention the ds gag RNA inhibits or down regulates p24 synthesis. The sense and antisense RNA molecules are expected to cause only a modest, if any, inhibitory effect on p24 synthesis, unless these RNAs were able to form some degree of double strandedness.

### EXAMPLE 2: DETERMINATION OF THE EXTENT OF REDUCTION OF P24 SYNTHESIS FROM ONE CELL CULTURE TO ANOTHER

To demonstrate that the down-regulated signal can be transmitted to cells which have not been down-regulated, this example demonstrates that the reduction/inhibition effect (i.e., inhibition or reduction of p24 synthesis) is transmitted to cells in culture that are not transfected by the agent.

### A. Co-Culture of COS 7 and RD cells

Cells from the cultures of Example 1 which demonstrate reduction of p24 synthesis are co-cultured with control cells of cells that have not previously been incubated with any RNA molecule, and are, in fact, synthesizing p24 at wild-type levels. According to the present invention, the previously transfected cells can transfer the target polynucleotide function inhibition to non-transfected cells, and the control cells in the co-culture are characterized by a reduction in synthesis of p24.

In order to distinguish control cells from the previously transfected cells in the culture, a first protocol is followed: The COS 7 cells of Example 1 which demonstrate inhibition of p24 synthesis are co-cultured with non-transfected RD cells expressing p24 at wildtype levels at various ratios of cell types, e.g., the ratios range from 1/1000 to 1/10 (COS 7/RD) to a total of 6 - 7 x10⁵ cells in 6 well plates. After 2 days of culture under the conditions specified in Example 1, the RD cells in the cultures are examined for p24 synthesis. The cells are examined about 3 times per week for 3 weeks.

p24 synthesis is assayed by two methods. In the first method, the media from the co-cultured cells is assayed for p24 using the p24 ELISA assay (Coulter). In the second method, cells are immunostained for p24 using rabbit polyclonal sera (Intracell Corp.) against p24 and anti-rabbit IgG conjugated to FITC. Because COS 7 and RD cells are distinguishable by morphology, a loss of stain in the RD cells can be readily distinguished from the COS 7 cells. Because COS 7 cells express T Antigen while RD cells do not, the co-cultured cells are also stained for T Ag using mouse monoclonal sera against SV40 T antigen (Pharmagen Corp.) and anti-mouse IgG conjugated to r-phycoerythrin (PE). Only the COS 7 cells stain under these conditions. The cell staining is determined by fluorescence microscopy or by FLOW cytometry.

The inhibition of p24 function in RD cells in coculture is demonstrated by comparison to a control culture containing only the RD cells by a loss of FITC stain in the co-cultured RD cells. RD cells in the coculture that are not stained with FITC or PE are evidence of reduction or inhibition of the p24 synthesis function of the p24 target polynucleotide by the RNA molecules (particularly the ds RNA molecules) of Example 1.

### B. Cultures of transfected RD cells with non-transfected RD cells

In a second protocol, the transfected RD cells of Example 1, which demonstrate reduced p24 production are co-cultured with non-transfected RD cells which are engineered to contain an integrated hygromycin resistance gene and express normal levels of p24 using different ratios of cells, with ratios ranging from 1/1000 to 1/10 (RD/control RD) to a total cell number of 6 - 7x10⁵ in a 6 well plate. Hygromycin-resistant RD cells are made as follows: RD cells (5-6x10⁵ cells) are cultured to 80-90% confluence in a six-well plate and are transfected with 2.5 µg of the Nru 1-Sal 1 fragment of pCEP4 (Invitrogen Corp.) that contains the hygromycin resistance gene under the control of a thymidine kinase (TK) promoter. Transfections are done using the transfecting agent, lipofectamine (Gibco BRL). Two days following transfection, the cells are incubated in the presence of 400 µg/ml hygromycin. Resistant cells are clonally expanded. One or more of the clonally expanded cell lines are used as the control in the experiment.

From 1 day to several weeks after co-culture under the conditions specified in Example 1, replicate co-cultures are incubated with 400 µg/ml hygromycin. This concentration of hygromycin kills the RD cells that are not hygromycin resistant, leaving only the control hygromycin resistant RD cells. The remaining resistant cells are derived from the control cells. P24 levels are measured directly from the control cells, for example using the ELISA of Example 1 as well as by immunostaining as above described.

According to the present invention, inhibition of p24 production is revealed in at least a subset of the control cells.

### EXAMPLE 3: - IN VIVO INHIBITION OF ENDOGENOUS INTERLEUKIN-12 PRODUCTION BY THE METHOD OF THIS INVENTION

### A. Design of RNA molecules as Compositions of the Invention

All RNA molecules in this experiment are close to 600 nts in length, and all RNA molecules are designed to be incapable of producing the p40 chain of IL-12. The molecules have no cap and no poly-A sequence; the native initiation codon is not present, and the RNA does not encode the full-length product. The following RNA molecules are designed:
(1) a single-stranded (ss) sense RNA polynucleotide sequence homologous to IL-12 p40 murine messenger RNA (mRNA);
(2) a ss anti-sense RNA polynucleotide sequence complementary to IL-12 p40 murine mRNA,
(3) a double-stranded (ds) RNA molecule comprised of both sense and anti-sense p40 IL-12 murine mRNA polynucleotide sequences,
(4) a ss sense RNA polynucleotide sequence homologous to IL-12 p40 murine heterogeneous RNA (hnRNA),
(5) a ss anti-sense RNA polynucleotide sequence complementary to IL-12 p40 murine hnRNA,
(6) a ds RNA molecule comprised of the sense and anti-sense IL-12 p40 murine hnRNA polynucleotide sequences,
(7) a ss murine RNA polynucleotide sequence homologous to the top strand of the IL-12 p40 promoter,
(8) a ss murine RNA polynucleotide sequence homologous to the bottom strand of the IL-12 p40 promoter, and
(9) a ds RNA molecule comprised of murine RNA polynucleotide sequences homologous to the top and bottom strands of the IL-12 p40 promoter.

As a negative control the sense, anti-sense and ds RNAs derived from the HSV2 gD gene described in Example 1 are also used. Another control group is composed of mice receiving no RNA.

As described in Example 1, the various RNA molecules of (1)-(9) above are generated through T7 RNA polymerase transcription of PCR products bearing a T7 promoter at one end. In the instance where a sense RNA is desired, a T7 promoter is located at the 5' end of the forward PCR primer. In the instance where an antisense RNA is desired, the T7 promoter is located at the 5' end of the reverse PCR primer. When dsRNA is desired both types of PCR products are included in the T7 transcription reaction. Alternatively, sense and anti-sense RNA are mixed together after transcription.

The PCR primers used in the construction of the RNA molecules of this Example are 5' to 3', with the top strand of the T7 promoter underlined.
Forward IL-12 genomic (hnRNA) [SEQ ID NO: 9]: 5' TCAGCAAGCACTTGCCAAACTCCTG 3' and Reverse IL-12 genomic (hnRNA) [SEQ ID NO: 10]: 5' GAGACAAGGTCTCTGGATGTTATTG 3';
T7 Forward IL-12 genomic (hnRNA) [SEQ IDNO: 11]: 5' GTAATACGACTCACTATAGGGTCAGCAAGCACTTGCCAAACTCCTG 3'
and T7 Reverse IL-12 genomic (hnRNA) [SEQ ID NO: 12]: 5' GTAATACGACTCACTATAGGGGAGACAAGGTCTCTGGATGTTATTG 3';
T7 Forward IL-12 promoter [SEQ ID NO: 13]: 5' GTAATACGACTCACTATAGGGCCTATAAGCATAAGAGACGCCCTC 3' and
Forward IL-12 promoter [SEQ ID NO: 14]: 5' CCTATAAGCATAAGAGACGCCCTC 3';
Reverse IL-12 promoter [SEQ ID NO: 15]: 5' GGCTGCTCCTGGTGCTTATATAC 3'
and T7 Reverse IL-12 promoter [SEQ ID NO: 16]: 5' GTAATACGACTCACTATAGGGGGCTGCTCCTGGTGCTTATATAC 3';
T7 Forward IL-12 cDNA (mRNA) [SEQ ID NO: 17]: 5' GTAATACGACTCACTATAGGGTGTGTCCTCAGAAGCTAACCATC 3' and
Forward IL-12 cDNA (mRNA) [SEQ ID NO: 18]: 5' TGTGTCCTCAGAAGCTAACCATC 3';
Reverse IL-12 cDNA (mRNA) [SEQ ID NO: 19]: 5' GCAGGTGACATCCTCCTGGCAGGA 3'
and T7 Reverse IL-12 cDNA (mRNA) [SEQ ID NO: 20]: 5' GTAATACGACTCACTATAGGGGCAGGTGACATCCTCCTGGCAGGA 3'.

The genomic and PCR primer coordinates are based on the map supplied in the following citation: Tone et al, Eur. J. Immunol., 26:1222-1227 (1996). The forward IL-12 genomic primer maps to coordinates 8301-8325. The reverse IL-12 genomic primer maps to coordinates 8889-8913. The forward IL-12 promoter primer maps to coordinates 83-106. The reverse IL-12 promoter primer maps to coordinates 659-682. The coordinates for the cDNA PCR primers is based on GenBank Accession No. M86671. The forward IL-12 cDNA primer maps to coordinates 36-58. The reverse IL-12 cDNA primer maps to coordinates 659-682.

### B. Assay

Balb/c mice (5 mice/group) are injected intramuscularly or intraperitoneally with the murine IL-12 p40 chain specific RNAs described above or with controls identified above at doses ranging between 10 µg and 500 µg. Sera is collected from the mice every four days for a period of three weeks and assayed for IL-12 p40 chain levels using the Quantikine M-IL-12 p40 ELISA Assay (Genzyme).

According to the present invention, mice receiving ds RNA molecules derived from both the IL-12 mRNA, IL-12 hnRNA and ds RNA derived from the IL-12 promoter demonstrate a reduction or inhibition in IL-12 production. A modest, if any, inhibitory effect is observed in sera of mice receiving the single stranded IL-12 derived RNA molecules, unless the RNA molecules have the capability of forming some level of double-strandedness. None of the HSV gD derived RNAs are expected to reduce or inhibit IL-12 *in vivo* in a specific manner..

### EXAMPLE 4: METHOD OF THE INVENTION IN THE PROPHYLAXIS OF DISEASE

### A. In Vitro Assay

Vero and/or BHK cells, seeded at a density of 20-30% confluency, are cultured in six-well plates at 37°C in DMEM with 10% FBS. When cells are 80-90% confluent, they are transfected with 2-3 µg of the HIV gag- and HSV gD- specific RNA molecules described in Example 1 using lipofectamine (Gibco-BRL) as a transfecting agent. The RNA molecules are also delivered in the absence of any known transfecting agent in amounts varying between 5 and 100 µg. Another group of cells receives no RNA.

Still other groups of Vero and/or BHK cells are similarly transfected with 2-3 µg of a double-stranded DNA plasmid, plasmid 24, which is described in U. S. Patent No. 5,851,804, which contains a sequence encoding the HSV2 gD protein under the control of the HCMV promoter and a SV40 poly A sequence.

The transfected cells are cultured at 37°C in DMEM with 10% FBS. At days 1, 2, 4 and 7 following transfection, cells are infected with HSV2 at a multiplicity of infection (MOI) of 0.1 in an inoculum of 250 µl DMEM. The inoculum is allowed to adsorb for 1 hour after which 2 mls of DMEM (10% FBS) is added per well. For those cells infected at 4 and 7 days post transfection, the cells are passaged into a new six-well plate such that they are confluent at the time of infection. If the cells are not passaged, they become overcrowded.

At 36-48 hours post-infection, the cell lysates are assayed for viral titer by conventional plaque assay on Vero cells [Clinical Virology Manual, 2d edit., eds. S. Specter and G. Lancz, pp. 473-94 (1992)]. According to this invention, the cells transfected with the ds DNA plasmid, APL-400-024, and with the ds RNA molecule containing a polynucleotide sequence of the gD2 antigen, cannot be productively infected with HSV2. All other cells are anticipated to become productively infected with HSV2.

### B. In Vivo Assay

Using the HSV-gD specific RNA molecules described in Example 1, which do not have the ability to make HSVgD protein and HIV gag specific RNA molecules as controls, mice are evaluated for protection from HSV challenge through the use of the injected HSVgD specific RNA molecules of the invention.

Balb/c mice (5 mice/group) are immunized intramuscularly or intraperitoneally with the described RNA molecules at doses ranging between 10 and 500 µg RNA. At days 1, 2, 4 and 7 following RNA injection, the mice are challenged with HSV-2 (10⁵ pfu in 30 µls) by intravaginal inoculation. Everyday post HSV-2 inoculation, the mice are observed for signs of infection and graded on a scale of 0-4. Zero is no sign of infection; 1 denotes redness; 2 denotes vesicles and redness; 3 denotes vesicles, redness and incontinence; and 4 denotes paralysis.

According to the present invention, because the mice that receive dsRNA molecules of the present invention which contain the HSV gD sequence are shown to be protected against challenge. The mice receiving the HIV gag control RNA molecules are not protected. Mice receiving the ss RNA molecules which contain the HSV gD sequence are expected to be minimally, if at all, protected, unless these molecules have the ability to become at least partially double stranded *in vivo.* According to this invention, because the dsRNA molecules of the invention do not have the ability to make HSV gD protein, the protection provided by delivery of the RNA molecules to the animal is due to a non-immune mediated mechanism that is gene specific.
## Claims

1. A composition for use in a therapeutic method of treatment comprising inhibiting the function of a target polynucleotide sequence in a mammalian cell, wherein the target polynucleotide sequence is a non-coding sequence of a target gene, said composition comprising an agent that provides to a cell an at least partially double-stranded RNA molecule, wherein said at least partially double-stranded RNA molecule is less than about 1,000 nucleotides in length and contains a double stranded sequence of at least 11 to 30 nucleotides, wherein said at least partially double stranded RNA comprises a sense sequence of a target polynucleotide and an antisense sequence thereto and wherein said target gene is a naturally occurring mammalian sequence or a heterologous sequence present due to a mammalian intracellular pathogenic infection.

2. The composition for use according to claim 1, wherein said agent comprises one or more vectors comprising a promoter sequence operably linked to polynucleotides encoding sense and antisense strands of said at least partially dsRNA molecule.

3. The composition for use according to claim 2, wherein said sense and antisense strands of the at least partially dsRNA are encoded on separate vectors.

4. The composition for use according to claim 2, wherein said sense and antisense strands of the at least partially dsRNA are encoded on the same DNA vector.

5. The composition for use according to claim 2, wherein the vector is a DNA vector and said promoter sequence operably linked to the polynucleotides is a RNA pol III promoter sequence.

6. The composition for use according to claim 3, wherein said one or more vectors are plasmids.

7. The composition for use according to claim 3, wherein said one or more vectors are viral vectors.

8. The composition for use according to claim 1, wherein said target gene is a viral gene, wherein the viral gene is necessary for replication and/or pathogenesis of the virus in an infected mammalian cell.

9. The composition for use according to claim 1, wherein said target gene is a gene expressing an aberrant protein product.

## Patentansprüche

1. Zusammensetzung zur Verwendung in einem therapeutischen Behandlungsverfahren, das das Hemmen der Funktion einer Zielpolynucleotidsequenz in einer Säugetierzelle umfasst, wobei die Zielpolynucleotidsequenz eine nicht kodierende Sequenz eines Zielgens ist, wobei die Zusammensetzung ein Mittel umfasst, das einer Zelle ein zumindest teilweise doppelsträngiges RNA-Molekül bereitstellt, wobei das zumindest teilweise doppelsträngige RNA-Molekül weniger als etwa 1.000 Nucleotide lang ist und eine doppelsträngige Sequenz von zumindest 11 bis 30 Nucleotiden enthält, wobei die zumindest teilweise doppelsträngige RNA eine Sense-Sequenz eines Zielpolynucleotids und eine Antisense-Sequenz dazu umfasst und wobei das Zielgen eine natürlich vorkommende Säugetiersequenz oder eine heterologe Sequenz ist, die aufgrund einer intrazellulären pathogenen Infektion in Säugetierzellen vorliegt.

2. Zusammensetzung zur Verwendung nach Anspruch 1, wobei das Mittel einen oder mehrere Vektoren umfasst, die eine Promotorsequenz umfassen, die mit für Sense- und Antisense-Stränge des zumindest teilweise doppelsträngigen RNA-Moleküls kodierenden Polynucleotiden operabel verbunden ist.

3. Zusammensetzung zur Verwendung nach Anspruch 2, wobei die Sense- und Antisense-Stränge der zumindest teilweise doppelsträngigen RNA auf separaten Vektoren kodiert sind.

4. Zusammensetzung zur Verwendung nach Anspruch 2, wobei die Sense- und Antisense-Stränge der zumindest teilweise doppelsträngigen RNA auf demselben DNA-Vektor kodiert sind.

5. Zusammensetzung zur Verwendung nach Anspruch 2, wobei der Vektor ein DNA-Vektor ist und die Promotorsequenz, die mit den Polynucleotiden operabel verbunden ist, eine RNA-pol-III-Promotorsequenz ist.

6. Zusammensetzung zur Verwendung nach Anspruch 3, wobei der eine oder die mehreren Vektoren Plasmide sind.

7. Zusammensetzung zur Verwendung nach Anspruch 3, wobei der eine oder die mehreren Vektoren virale Vektoren sind.

8. Zusammensetzung zur Verwendung nach Anspruch 1, wobei das Zielgen ein Virusgen ist, wobei das Virusgen für die Replikation und/oder Pathogenese des Virus in einer infizierten Säugetierzelle notwendig ist.

9. Zusammensetzung zur Verwendung nach Anspruch 1, wobei das Zielgen ein Gen ist, das ein abweichendes Proteinprodukt exprimiert.

## Revendications

1. Composition destinée à être utilisée dans un procédé de traitement thérapeutique comprenant l'inhibition de la fonction d'une séquence de polynucléotide cible dans une cellule de mammifère, dans laquelle la séquence de polynucléotide cible est une séquence non codante d'un gène cible, ladite composition comprenant un agent qui fournit à une cellule une molécule d'ARN au moins partiellement double brin, où ladite molécule d'ARN au moins partiellement double brin possède une longueur inférieure à environ 1 000 nucléotides et contient une séquence double brin d'au moins 11 à 30 nucléotides, où ledit ARN au moins partiellement double brin comprend une séquence sens d'un polynucléotide cible et une séquence antisens à celle-ci et où ledit gène cible est une séquence de mammifère survenant à l'état naturel ou une séquence hétérologue présente en raison d'une infection pathogène intracellulaire chez le mammifère.

2. Composition destinée à être utilisée selon la revendication 1, dans laquelle ledit agent comprend un ou plusieurs vecteurs comprenant une séquence de promoteur en liaison fonctionnelle avec des polynucléotides codant pour les brins sens et antisens de ladite molécule d'ARN au moins partiellement double brin.

3. Composition destinée à être utilisée selon la revendication 2, dans laquelle lesdits brins sens et antisens de l'ARN au moins partiellement double brin sont codés sur des vecteurs distincts.

4. Composition destinée à être utilisée selon la revendication 2, dans laquelle lesdits brins sens et antisens de l'ARN au moins partiellement double brin sont codés sur le même vecteur d'ADN.

5. Composition destinée à être utilisée selon la revendication 2, dans laquelle le vecteur est un vecteur d'ADN et ladite séquence de promoteur en liaison fonctionnelle avec les polynucléotides est une séquence de promoteur de l'ARN pol III.

6. Composition destinée à être utilisée selon la revendication 3, dans laquelle lesdits un ou plusieurs vecteurs sont des plasmides.

7. Composition destinée à être utilisée selon la revendication 3, dans laquelle lesdits un ou plusieurs vecteurs sont des vecteurs viraux.

8. Composition destinée à être utilisée selon la revendication 1, dans laquelle ledit gène cible est un gène viral, où le gène viral est nécessaire pour la réplication et/ou la pathogenèse du virus dans une cellule de mammifère infectée.

9. Composition destinée à être utilisée selon la revendication 1, dans laquelle ledit gène cible est un gène exprimant un produit protéique aberrant.


<110> American Home Products Corporation Pachuk, Catherine Satishchandran, C.
<120> Methods and Compositions for Inhibiting the Function of Polynucleotide Sequences
<130> AHP28APCT
<140>
   <141>
<150> 60/130,377
   <151> 1999-04-21
<160> 20
<170> PatentIn Ver. 2.1
<210> 1
   <211> 47
   <212> DNA
   <213> Artificial Sequence
<220>
   <223> Description of Artificial Sequence: T7 forward gag primer
<400> 1
   gtaatacgac tcactatagg gcggcaggga gctagaacga ttcgcag 47
<210> 2
   <211> 25
   <212> DNA
   <213> Artificial Sequence
<220>
   <223> Description of Artificial Sequence: reverse gag primer
<400> 2
   ctgctatgtc acttcccctt ggttc 25
<210> 3
   <211> 48
   <212> DNA
   <213> Artificial Sequence
<220>
   <223> Description of Artificial Sequence: T7 reverse gag primer
<400> 3
   gtaatacgac tcactatagg gcgctgctat gtcacttccc cttggttc 48
<210> 4
   <211> 24
   <212> DNA
   <213> Artificial Sequence
<220>
   <223> Description of Artificial Sequence: forward gag primer
<400> 4
   gcagggagct agaacgattc gcag 24
<210> 5
   <211> 47
   <212> DNA
   <213> Artificial Sequence
<220>
   <223> Description of Artificial Sequence: T7 forward gD primer
<400> 5
   gtaatacgac tcactatagg gcggtcgcgg tgggactccg cgtcgtc 47
<210> 6
   <211> 24
   <212> DNA
   <213> Artificial Sequence
<220>
   <223> Description of Artificial Sequence: forward gD primer
<400> 6
   gtcgcggtgg gactccgcgt cgtc 24
<210> 7
   <211> 47
   <212> DNA
   <213> Artificial Sequence
<220>
   <223> Description of Artificial Sequence: T7 reverse gD primer
<400> 7
   gtaatacgac tcactatagg gcggtgatct ccgtccagtc gtttatc 47
<210> 8
   <211> 24
   <212> DNA
   <213> Artificial Sequence
<220>
   <223> Description of Artificial Sequence: reverse gD primer
<400> 8
   gtgatctccg tccagtcgtt tatc 24
<210> 9
   <211> 25
   <212> DNA
   <213> Artificial Sequence
<220>
   <223> Description of Artificial Sequence: forward IL-12 genomic
<400> 9
   tcagcaagca cttgccaaac tcctg 25
<210> 10
   <211> 25
   <212> DNA
   <213> Artificial Sequence
<220>
   <223> Description of Artificial Sequence: reverse IL-12 genomic
<400> 10
   gagacaaggt ctctggatgt tattg 25
<210> 11
   <211> 46
   <212> DNA
   <213> Artificial Sequence
<220>
   <223> Description of Artificial Sequence: T7 forward IL-12 genomic
<400> 11
   gtaatacgac tcactatagg gtcagcaagc acttgccaaa ctcctg 46
<210> 12
   <211> 46
   <212> DNA
   <213> Artificial Sequence
<220>
   <223> Description of Artificial Sequence: T7 reverse IL-12 genomic
<400> 12
   gtaatacgac tcactatagg ggagacaagg tctctggatg ttattg 46
<210> 13
   <211> 45
   <212> DNA
   <213> Artificial Sequence
<220>
   <223> Description of Artificial Sequence: T7 forward IL-12 primer
<400> 13
   gtaatacgac tcactatagg gcctataagc ataagagacg ccctc 45
<210> 14
   <211> 24
   <212> DNA
   <213> Artificial Sequence
<220>
   <223> Description of Artificial Sequence: forward IL-12 promoter
<400> 14
   cctataagca taagagacgc cctc 24
<210> 15
   <211> 23
   <212> DNA
   <213> Artificial Sequence
<220>
   <223> Description of Artificial Sequence: reverse IL-12 promoter
<400> 15
   ggctgctcct ggtgcttata tac 23
<210> 16
   <211> 44
   <212> DNA
   <213> Artificial Sequence
<220>
   <223> Description of Artificial Sequence: T7 reverse IL-12 promoter
<400> 16
   gtaatacgac tcactatagg gggctgctcc tggtgcttat atac 44
<210> 17
   <211> 44
   <212> DNA
   <213> Artificial Sequence
<220>
   <223> Description of Artificial Sequence: T7 forward IL-12 cDNA
<400> 17
   gtaatacgac tcactatagg gtgtgtcctc agaagctaac catc 44
<210> 18
   <211> 23
   <212> DNA
   <213> Artificial Sequence
<220>
   <223> Description of Artificial Sequence: forward IL-12 cDNA
<400> 18
   tgtgtcctca gaagctaacc atc 23
<210> 19
   <211> 24
   <212> DNA
   <213> Artificial Sequence
<220>
   <223> Description of Artificial Sequence: reverse IL-12 cDNA
<400> 19
   gcaggtgaca tcctcctggc agga 24
<210> 20
   <211> 45
   <212> DNA
   <213> Artificial Sequence
<220>
   <223> Description of Artificial Sequence: T7 reverse IL-12 cDNA
<400> 20
   gtaatacgac tcactatagg ggcaggtgac atcctcctgg cagga 45